# EUROPEAN PATENT SPECIFICATION

(11) **EP 1 534 376 B1**
(45) Date of publication and mention of the grant of the patent: **31.08.2016**
(21) Application number: 03761282.7
(22) Date of filing: 19.06.2003
(51) Int. Cl.: A61M 31/00, A61M 37/00, A61K 9/00, A61K 9/70

(54) **RAPIDLY DISSOLVING MICRO-PERFORATOR FOR DRUG DELIVERY AND OTHER APPLICATIONS**
SICH SCHNELL AUFLÖSENDER MIKROPERFORATOR FÜR DIE MEDIKAMENTENABGABE UND ANDERE ANWENDUNGEN
MICROPERFORATEUR A DISSOLUTION RAPIDE POUR ADMINISTRATION DE PRODUITS PHARMACEUTIQUES ET AUTRES APPLICATIONS

(30) Priority: 25.06.2002 US 179749; 16.09.2002 US 411063 P
(43) Date of publication of application: 01.06.2005
(73) Proprietor: Theraject, Inc., Fremont, CA 94538 (US)
(72) Inventor: Kwon, Sung-Yun, Fremont, CA 94536 (US)
(74) Representative: Prüfer & Partner mbB Patentanwälte · Rechtsanwälte
(86) International application number: PCT/US2003/019820
(87) International publication number: WO 2004/000389

(56) References cited:
- WO-A1-02/05889
- WO-A2-02/064193
- US-A- 5 749 376
- US-A- 6 050 988
- US-A- 6 083 196
- US-A- 6 132 755
- US-A1- 2002 082 543
- US-B1- 6 334 856
- US-B1- 6 611 707

## Description

### Field of the Invention

This invention relates to percutaneous delivery of drugs.

### Background of the Invention

To be effective any drug must be transported across one or more biological barriers in the body, at rates and in amounts that are therapeutically effective. In many instances drugs can be administered orally (*per os*) with sufficient efficacy, but in many other instances the oral route is not effective because of severe degradation in the gastrointestinal tract, poor absorption in intestinal membrane, and/or first pass breakdown by the liver. These challenges are particularly problematic for many of the newer drugs, including proteins, peptides, and DNA constituents. Recombinant human insulin, growth hormone, erythropoietin, interferon and other proteins, for example, must all currently be administered in some manner other than orally.

An alternative is to deliver drugs percutaneously, i.e., through or across the skin. Typically, this is accomplished using parental injection with standard syringes or catheters. Unfortunately, needle injection can provoke needle phobia, substantial pain, and local damage to the skin in many patients. Needle injection is also problematic for continuous delivery of a drug, or for continuous diagnosis.

One solution to these problems is to administer drugs transdermally, which usually involves diffusion of a drug across the skin. Once a drug reaches the dermal depth (below the epidermal layer), the drug diffuses rapidly to deep tissue layers and other parts of the system via blood circulation. But the outermost layer of skin, the stratum corneum, represents a major barrier to transdermal drug penetration, so that the poor skin permeability of many drugs militates against the broad applicability of transdermal delivery.

Chemical enhancers, iontophoresis, electroporation, ultrasound, and heat elements have all been used to improve transdermal delivery. However, these techniques are not suitable for some types of drugs, and often fail to provide a therapeutic level of delivery. Moreover, these techniques sometimes result in undesirable skin reactions, and/or are impractical for continuous controlled drug delivery over a period of hours or days.

Some attempts have been made to improve transdermal delivery using particle or liquid injection. A main advantage of those techniques is elimination of needles, and reduction of incidence of contamination. However, liquid injection frequently causes pain and/or sub-dermal hemorrhage. One technique, ballistic particle injection, is difficult to administer precisely and continuously, and can cause micro bleeding.

Others have used micro-needles (< 1mm in diameter) to effect percutaneous drug delivery. Micro-needles have been used to deliver a drug through a lumen in the needles, to deliver a drug along the outside of the needle shafts, or as skin perforators for subsequent patch drug application. Silicon micro-needles, for example, have been developed using fabrication procedures from the semiconductor industry. Examples are described in US 6334856 to Allen et al. (Jan. 2001), US 6256533 to Yuzhakov, et al. (July 2001), US 6312612 to Sherman, et al., (Nov. 2001), and US 6379324 to Gartstein, et al. (April 2002). Unfortunately, silicon needles are not dissolvable in the skin, and when broken during use can produce considerable irritation and even infection.

Dissolvable implants are known, but only in slowly dissolving formulations. Examples are US 6485453 to Buch-Rasmussen et al. (Nov 2002), US 5021241 to Yamahira et al. (June 1991), and WIPO WO/9608289 to Societe de Conseils de Recherché et d'Applications Scientifiques, S.A. (Publ March 1996). As a result, the use of dissolvable implants has largely been limited to hormones and other substances in which it is desirable to administer low doses over extended periods of time.

A corollary is that there has been no incentive to produce arrays of micro-needles or other micro implantables. As long as the product is being slowly dissolved, administration is better controlled using a single micro-needle to deliver small doses, or a single larger needle or pellet to deliver greater doses. (See e.g. US 4077406 to Sandhage et al., (March 1978).

Rapidly dissolving micro-implants would be desirable because they would allow administration of intermediate doses with only minimal pain and discomfort. But that concept has heretofore been difficult to achieve due to the absence of vascularity in the stratum corneum and epidermis, which prevents controlled dissolution of the micro-implants. Thus, there remains a need to develop rapidly dissolving percutaneous drug delivery vehicles, including especially arrays of micro-needles or other micro-perforators of skin.

Furthermore, document US 5 749 376 A discloses a medical method for decontamination of used surgical instrumentation made of biocompatible material.

### Summary Of The Invention

At least one problem mentioned above is solved by a skin patch device according to claim 1. Further developments are subject of the dependent claims.

Apparatus and methods are described that deliver drugs percutaneously using rapidly dissolving micro-perforators ("RDMP"). As used herein the term "micro-perforators" refers generically to needles, blades, cutting, puncturing, or any other devices measuring less than 1mm in average diameter, regardless of cross-sectional shape.

Micro-perforators include a solid matrix of dissolvable (including meltable and biodegradable) material that holds one or more selected drugs. The drug(s) within the matrix material can be distributed interstitially or in any other suitable manner. Micro-perforators can be fluidly associated with a reservoir of the same or different drug, which drug can be carried into the skin by the microneedles themselves, or by channels created by perforation of the skin by the microneedles. In preferred embodiments the reservoir includes a solvent that speeds the dissolving of the micro-perforators following application of the micro-perforators to the skin. The reservoir can advantageously be included in a patch, which can include a ring of adhesive that bonds with, and holds the reservoir against, the patient's skin overlying the perforated region of skin.

Inclusion of swelling materials within the matrix facilitates creation of drug transport channels by keeping the incisions open for a longer period of time. Such channels, especially in the outermost layer of skin, can advantageously diminish the barrier properties of skin for controlled drug delivery, or for providing access to monitor body fluids or other analytes.

Micro-perforators are contemplated herein to include either a single drug or a mixture of drugs, depending upon the application and physical/chemical properties of the drug(s). The micro-perforators dissolve rapidly, which is defined herein to mean that at least 50% of the micro-perforators dissolve in or on the skin within 1 hour of administration.

Various objects, features, aspects and advantages of the present invention will become more apparent from the following detailed description of preferred embodiments of the invention, along with the accompanying drawings in which like numerals represent like components.

### Brief Description of the Drawing

Figure 1 is a schematic cross-section of a patient's skin.
Figures 2A-2G are perspective views of examples of suitable micro-perforators.
Figure 3 is a plan view of an array of micro-perforators and a surrounding annular ring of adhesive that are part of a RDMP system.
Figures 4-9 illustrate activation mechanisms that can be used for RDMP skin penetration.
Figure 10 illustrates a drug reservoir system.
Figures 11A-11C and 12A-12F illustrate operation of some contemplated patch systems.

### Description of Best Modes of the Invention

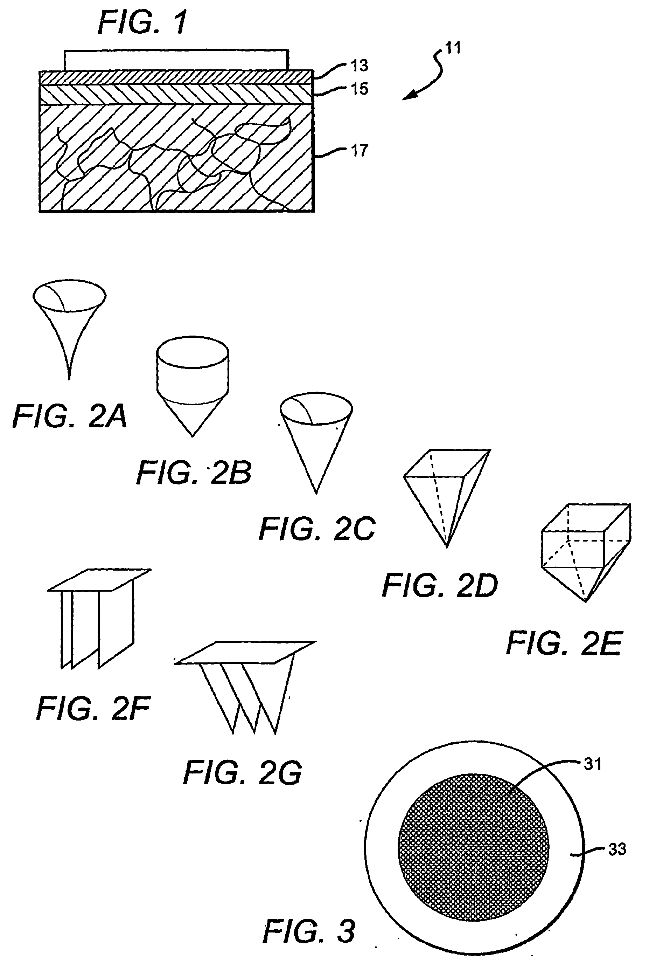

**Figure 1** is a cross-sectional view of the top layers of the skin 11, including a stratum corneum 13, an epidermal layer or epidermis 15 and a dermal layer or dermis 17. The outermost layer of skin, the stratum corneum 13, is a dead cell layer, usually between 10 and 20 microns (*µ*m) thick. The stratum corneum 13 contains hydrophilic keratinocytes surrounded by a hydrophobic extra-cellular matrix of lipids, mainly ceramide. Due to the structural and compositional uniqueness, the stratum corneum 13 presents the greatest barrier to transdermal flux of drugs or other molecules into the body, and of body fluids and other analytes out of the body. The stratum corneum 13 is continuously renewed by shedding of corneum cells, with an average turnover time of 2-3 weeks.

Below the stratum corneum 13 is the viable epidermis or epidermal layer 15, which is between 50 and 100 *µ*m thick. The epidermis contains no blood vessels and freely exchanges metabolites by diffusion to and from the dermis 17, located immediately below the epidermis 15. The dermis is between 1 and 3 mm thick and contains blood vessels, lymphatics, and nerves. Once a drug reaches the dermal layer, the drug will perfuse through system circulation.

Preferred drug delivery systems include an array of rapidly dissolving micro-perforators, preferably at least 5, 10, 25, 50, 100 or even 200 micro-perforators in an area of about 1 cm², at least one or which is formed as a solid matrix of one or more needles or blades, each pointed or sharpened at a first end for perforation of the skin. Each micro-perforator is strong and intact enough to pierce the stratum corneum, and is either biodegradable or dissolvable when the micro-perforator (and drug) has penetrated into the patient's body with body fluid and/or solvent in the drug reservoir. The biodegradation or dissolution process may advantageously occur over a desired time interval of between a few tens of seconds and a few hours.

The present inventors have discovered that desired dissolution rates can be effectively accomplished by providing an external source of solvent, and that such solvent can be conveniently provided by an overlying patch. Many contemplated micro-perforators have been demonstrated in which a patch dispenses water as the main solvent, along with a drug. The micro-perforators can be a matrix containing a solid solution of the same or a different drug, and depending on the composition of the matrix, and size and shape of the micro-perforators, at least 50% of the micro-perforators can be made to dissolve within any desired time period of from tens of seconds, to minutes, and to one or more hours of application.

**Figures 2A-2G** are perspective views of some of the contemplated shapes of micro-perforators used in an array. The micro-perforator in **Figure 2A** is a cone with a cusp-like point that may provide extra penetration power. The micro-perforator in **Figure 2C** is a conventional cone shape with a conventional linear penetration point. The micro-perforators in **Figures 2B and 2E** are cylinder-plus-cone shape for a circular cylinder and for a rectangular (or, more generally, polygonal) cylinder, respectively. The micro-perforator in **Figure 2D** is a polygonal cone shape. The micro-perforators in **Figures 2F and 2G** provide sequences of one or more sharp or slicing blades (straight or serrated) that contact and penetrate the skin. Other shapes with a pointed or blade end can also be used here.

Micro-perforators can have any suitable shape, including by way of example straight or tapered shafts, pyramids, wedges, or blades as illustrated in Figures 2A-2G. In a preferred embodiment, the outer diameter of a micro-perforator is greatest at the base or second end, about 1-1000 *µ*m, and the micro-perforator outer diameter near the first end is preferably 5-500 *µ*m, and more preferably 5-25 *µ*m at the first end. Micro-perforators can advantageously have a minimum tip diameter of no more than 20 *µ*m. The length of a micro-perforator is typically in a range 1-2000 *µ*m, more preferably in a range 100 - 1000 *µ*m. The skin is not a smooth and rugged surface and has different depths microscopically. In addition, the thickness of the stratum corneum and elasticity of the skin varies from person to person and from location to location on any given person's body. A desirable penetration depth has a range, rather than a single value, for effective drug delivery and relatively painless and bloodless penetration. Penetration depth of a micro-perforator can affect pain as well as delivery efficiency. In transdermal applications, the "penetrated depth" of the Micro-perforator is preferably less than 100 *µ*m so that a micro-perforator, inserted into the skin through the stratum corneum, does not penetrate past the epidermis. This is an optimal approach to avoid contacting nerves and blood vessels. In such applications, the actual length of the micro-perforator can be longer to account for the basal layer associated with the RDMP system not being fully inserted into the skin due to excessive elasticity and/or roughness of the skin.

Depending upon medical needs, micro-perforator penetration to the dermis may be required in some applications. In these instances, the penetrating portion of a micro-perforator can be optimized by adjusting several variables (length, dimension, mechanical properties of basal or substrate layer as well as stroke and speed of insertion of a micro-perforator), as well as accounting for target skin elasticity, skin hardness and surface roughness.

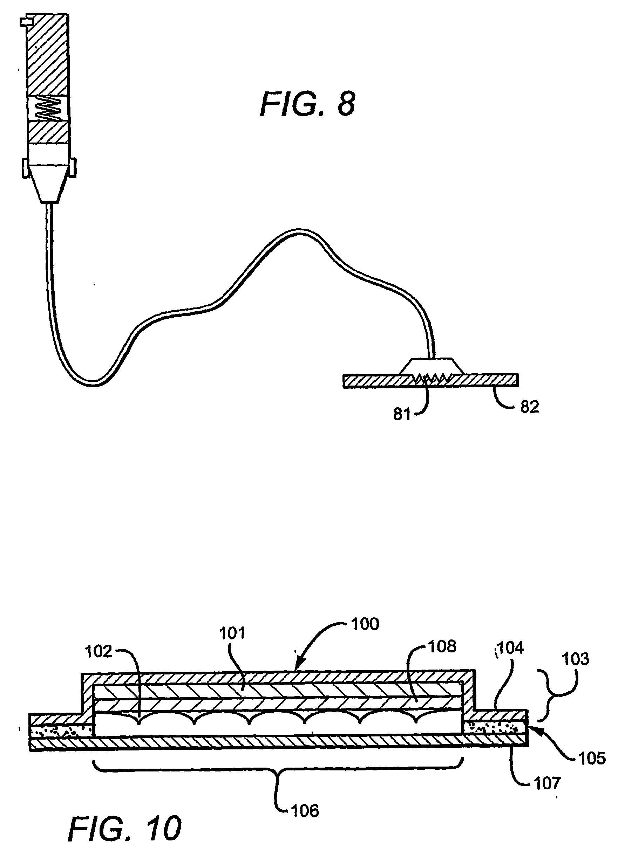

A basal layer (shown in Figures 3 and 10) provides protection to isolate a perforated skin region from contamination, and the basal layer can contain anti-bacterial agents and can create occlusion to hydrate perforated skin to enhance flux of fluids. A portal channel likely will contract or expand depending on the micro-perforator material properties after the micro-perforator dissolves or swells.

The primary functions of a micro-perforator are to pierce the stratum corneum, to provide prompt initiation and cut-off of drug delivery, and optionally to help keep the channel open for subsequent drug delivery or body fluid monitoring. As long as a micro-perforator dissolves reasonably quickly (which depends upon the circumstances) and is strong enough to pierce the stratum corneum, any biocompatible material can serve as a micro-perforator.

In producing a micro-perforator, a mold can be prepared using precision machining, micro-machining (such as MEMS), or laser-based or electro-discharge machining. Once the mold is prepared, a liquid solution, including the matrix material and the selected drug(s), is cast in the mold and dried. Depending on the viscosity and other physical and chemical properties of the liquid solution, additional force such as centrifuge force or compression force may be needed to fill the mold. To form a solid solution, the solvent needs to be air-dried, vacuum-dried or freeze-dried. Once a solid solution is formed, a micro-perforator is separated from the mold and cut to an appropriate shape and size.

Where a powder form is used for the matrix material, the powder can advantageously be spread over the mold. Depending upon the chemical and physical properties of the powder, appropriate heating of the powder may then be applied to melt or insert viscous materials into the mold. Alternatively, the powder may be inserted into the mold by pressure and/or application of heating, with or without use of binding agents. When micro-perforators have been formed into an array, the array is cooled, separated from the mold, and incorporated into a RDMP system.

Another suitable approach for fabrication of an array is photo-cross linking. A polymer solution including a photo-initiator is cast on the mold, and is solidified by irradiation. Once the solution is solidified, the solid solution can be peeled off and cut and shaped to appropriate size. Another feasible approach for making arrays of micro-perforators is fabrication of a plurality of micro-fibers that are spooled, arrayed and combined in a plunger.

A great many polymers are suitable matrix materials for micro-perforators, including but not limited to polyvinylpyrolidone (PVP), polyethylene glycol (PEG), polyvinyl alcohol (PVA), cellulose, hydroxypropyl cellulose (HPC), hydroxyethyl cellulose (HEC), hydroxypropyl methylcellulose (HPMC), dextrin, mono- and polysaccharide, sodium carboxymethyl cellulose, polyalcohol, gelatin, gum arabic, alginate, chitosan cylcodextrin and other biopolymers.

Carbohydrate derivatives, such as sugar derivatives (trehalose, glucose, maltose, lactose, lactulose, fructose, turanose, melitose, melezitose, dextran, sorbitol, xylitol, palatinit and mannitol) can be used. Water-soluble glasses, such as phosphate, nitrate and carboxylate glasses, as well as magnesium chloride, potassium chloride and calcium chloride can be also used for a matrix material, either alone or mixed with a matrix polymer.

Other examples of suitable matrix materials include non-ionic hydrophilic or ionic surfactants or lipophilic additives selected from among alkylglucosides, alkylmaltosides, alkylthioglucosides, lauryl macrogolglycerides, polyoxyethylene alkyl ethers, polyoxyethylene alkylphenols, polyethylene glycol fatty acids esters, polyethylene glycol glycerol fatty acid esters, polyoxyethylene sorbitan fatty acid esters, polyoxyethylene-polyoxypropylene block copolymers, polyglycerol fatty acid esters, polyoxyethylene glycerides, polyoxyethylene sterols, derivatives, and analogues thereof, polyoxyethylene vegetable oils, polyoxyethylene hydrogenated vegetable oils, reaction mixtures of polyols and at least one member of the group consisting of fatty acids, glycerides, vegetable oils, hydrogenated vegetable oils, and sterols, tocopherol polyethylene glycol succinates, sugar esters, sugar ethers; sucroglycerides, and mixtures thereof.

Suitable ionic surfactants include alkyl ammonium salts; bile acids and salts, analogues, and derivatives thereof; fatty acid derivatives of amino acids, carnitines, oligopeptides, and polypeptides; glyceride derivatives of amino acids, oligopeptides, and polypeptides; acyl lactylates; mono-diacetylated tartaric acid esters of mono-diglycerides; succinylated monoglycerides; citric acid esters of mono-diglycerides; alginate salts; propylene glycol alginate; lecithins and hydrogenated lecithins; lysolecithin and hydrogenated lysolecithins; lysophospholipids and derivatives thereof; phospholipids and derivatives thereof; salts of alkylsulfates; salts of fatty acids; sodium docusate; and mixtures thereof.

Suitable lipophilic additives include alcohols, polyoxyethylene alkylethers, fatty acids, bile acids, glycerol fatty acid esters, acetylated glycerol fatty acid esters, lower alcohol fatty acids esters, polyethylene glycol fatty acids esters; polyethylene glycol glycerol fatty acid esters, polypropylene glycol fatty acid esters, polyoxyethylene glycerides, lactic acid derivatives of mono/diglycerides, propylene glycol diglycerides, sorbitan fatty acid esters, polyoxyethylene sorbitan fatty acid esters, polyoxyethylene-polyoxypropylene block copolymers, transesterified vegetable oils, sterols, sterol derivatives, sugar esters, sugar ethers, sucroglycerides, polyoxyethylene vegetable oils, polyoxyethylene hydrogenated vegetable oils, reaction mixtures of polyols and at least one member of the group consisting of fatty acids, glycerides, vegetable oils, hydrogentated vegetable oils, and sterols, and mixtures thereof.

As used herein, an "array" of micro-perforators means any grouping of at least 9 micro-perforators, regardless of whether or not they are regularly spaced apart. Typically, arrays will include many more micro-perforators, preferably a hundred or more. For manufacturing convenience the micro-perforators in an array will generally (but not necessarily) all be about the same size and shape, and all have substantially the same composition.

**Figure 3** is a plan view of a patch system formed over an array of micro-perforators 31 that are optionally surrounded by a basal layer including an annular region 33 containing adhesive and an anti-bacterial agent (optional), to isolate the perforation region from other regions on the patient's skin. The annular region 33 of adhesive is intended to hold the array to the skin and to prevent, or reduce the likelihood of, entry of foreign matter and/or external infection. Optionally, a drug reservoir (not shown in Figure 3) may be located above the micro-perforator array 31 and/or may also be enclosed by the annular region 33 of adhesive.

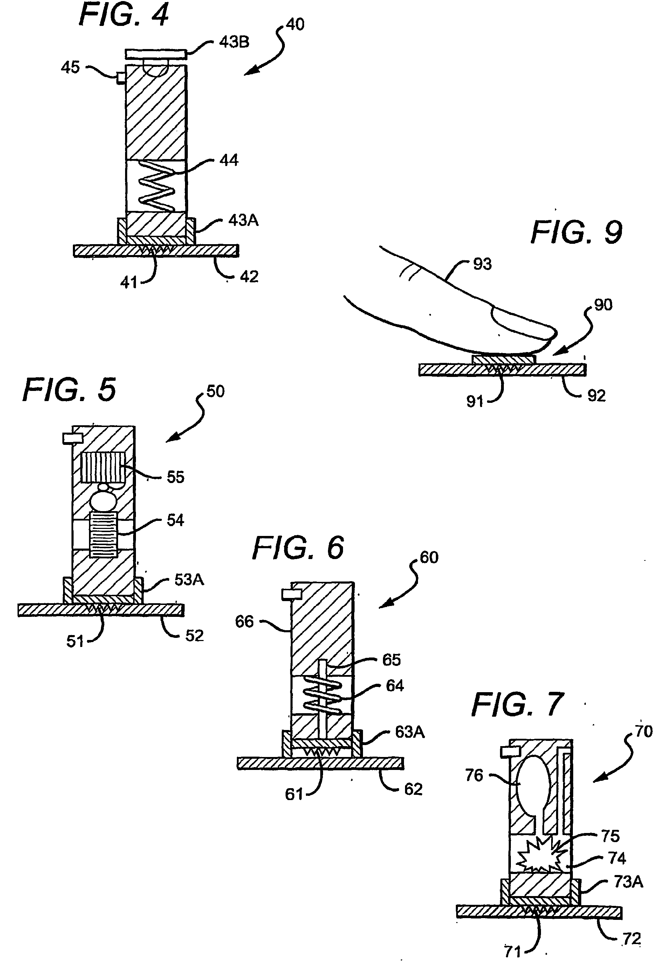

**Figure 4** is a sectional side view illustrating a perforation activation-deactivation mechanism 40 that causes one or more micro-perforators 41 to penetrate, or withdraw from, the patient's skin 42 to initiate and/or cut off delivery of a drug contained in the micro-perforator(s). Initiation or cut off of drug delivery can occur in as little as about 5 minutes, by insertion or removal of the Micro-perforator(s) 41. Depth of micro-perforator penetration is adjusted by a perforation adjustment mechanism, 43A and 43B (optional), such as a knob or screw. Micro-perforators 41 are driven into the patient's skin 42 by a spring or similar mechanism 44 that is controlled by a perforation mechanism trigger 45.

**Figure 5** is a sectional side view illustrating a perforation activation-deactivation mechanism 50 in which one or more Micro-perforators 51 is driven into, or withdrawn from, the patient's skin 52 by a screw 54 that is controlled by a screw knob or motor or similar device 55.

**Figure 6** is a sectional side view illustrating a perforation activation-deactivation mechanism 60 in which one or more Micro-perforators 61 is urged into the patient's skin 62 by a vacuum device 65, then injected by a spring 64 that causes the micro-perforators 62 to be extended from, or withdrawn into, an activation housing 66. Application of a mild vacuum in the skin perforation region may help to reduce skin variability.

**Figure 7** is a sectional side view illustrating a perforation activation-deactivation mechanism 70 in which one or more Micro-perforators 71 is driven into, or withdrawn from, the patient's skin 72 by a gas expansion mechanism 74 including a gas pressure chamber 75 and a gas reservoir 76.

**Figure 8** illustrates one means by which the micro-perforator mechanisms shown in Figures 4, 5, 6 and/or 7 may be used to control movement, including but not limited to penetration depth, of the corresponding Micro-perforators 82 into or out of the patient's skin 81. This design can be used with laparoscopes or endoscopes for internal drug delivery.

**Figure 9** is a sectional side view illustrating a perforation activation mechanism 90 in which one or more Micro-perforators 91 is driven into the patient's skin 92 by pressure, manually applied by a finger or other pressure mechanism 93, to one side of the micro-perforator(s).

Optionally, a drug patch system 100, illustrated in **Figure 10****,** includes a drug reservoir 101, containing a second drug that may be the same as or different from the first drug, that is located above and adjacent to the micro-perforator array 102 and that has an independently controlled reservoir drug delivery system 103. The drug patch system 100 preferably includes a backing film 104 that surrounds the drug reservoir 101 and includes an annular adhesive region 105 (basal layer, best illustrated in Figure 3) that surrounds and seals off the skin perforation region 106. A plastic release liner 107 is peeled off before skin perforation and protects the array until the liner is peeled off.

In a preferred embodiment, the reservoir in a patch system contains a drug and optionally contains a chemical enhancer in a liquid reservoir, a skin anti-irritant, an anti-infection chemical and/or other chemicals in liquid form. The chemical enhancer may be drawn from among alkyl alcohol, alpha bisabodol, decyl alcohol, dexpanthenol, dodecanol, ethylene glycol, fatty alcohols, glycerol, hexadecanol, isopropanol, octadecanol, tetrahydrofurfuryl alcohol, trichloroethanol, trifluoroethanol, alkyl acetamide, crotamiton, lauryl diethanolamide, toluamide, dimethyl acetamide, dimethyl formide, formamide, nicotinamide, acyl-amino-acids, alanine, arginine, proline, serine, aspartic acid, cysteine, glutamic acid, glycine, valine, leucine, isoleucine, protein aprotinin, azone, essential oils, such as carvone, cineole, eucalyptol, eugenol, methol, methone, terpene fatty acids, such as carboxyl acid, capric acid, diisopropyladipate, isopropyl myristate (IPM), isostearic acid, glyceryl monolaurate (GML), glycerol monooleate (GMO), lactic acid, linoleic acid, lauric acid, methyl laurate, methyl myristate, oleic acid, polyethylene glycol monolaurate, sorbitan monooleate (SMO), sucrose cocoate, sucrose monoloaurate, sucrose monooleate, triglyceride, macrocyclic enhancers, such as cyclodextrin, cyclopentadecanone and cyclopehtadecanolide, phospholipids, phospholipid/phosphate enhancers, such as dialkylphospahte, lecithin, dioxane, dioxolane, alkylsulfones, alkylsulfones, cetyl ether, cyclic dimethylsiloxane, decamethyltetrasiloxane, diallyl sulfoxides, dimethylsulfoxide, decylmethylsulfoxide, hexamethyldisiloxane, methyl octylsulfoxide, alkyl ammonium bromide, benzyl nicotinate, butylazocyclopentane, capsaicin, calcium thioglycolate, cyclic amine, diethyl sebacate, dimethylamino acetate, ethylene glycol monoethyl ether, imidazole, methylorthoformate, oxazoline, proline, urea, urethane, macrocyclis, amines, alkyl pyrolidones, N-methyl pyrrolidone, ethyl pyrrolidone, pyrrolidone, hydroxymethyl pyrrolidone, hexyl pyrrolidone, lauryl pyrrolidone, pyrrolidone-carboxylic acid, lauryl pyrrolidone carboxylic acid, pyroglutamic acid, sodium dedecyl sulfate, sodium deoxycholate, sodium lauryl sulfate, sorbitan monopalmitate, sorbitan trioleate, soybean casein, terpenes, piperazine derivatives, sodium traurocholate, liposome, bisbolol, dithiothreitol and vitamin E (□-tocopherol).

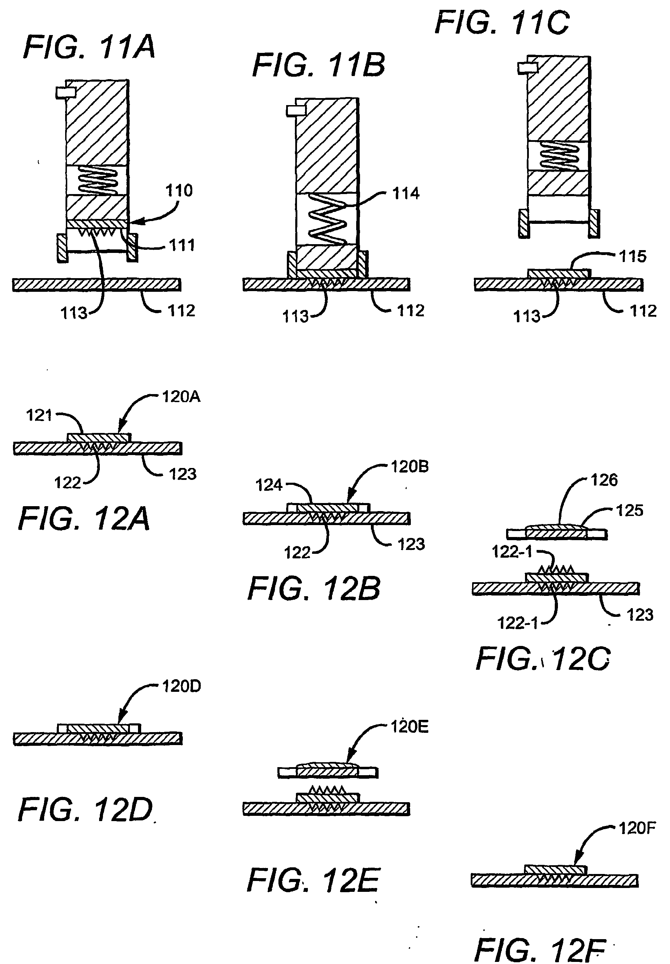

**Figures 11A/11B/11C** are sectional side views illustrating a preferred embodiment of a patch insertion process in a transdermal application. In **Figure 11A****,** adhesive in a basal layer 111 of a patch 110 is applied to the skin 112 to hold the patch against the skin. At this point, the micro-perforators 113 are displaced from the skin 112. In **Figure 11B**, a plunger or other mechanism 114 is activated to move the micro-perforators 113 into contact with the skin 112, to penetrate the stratum corneum and to enter the epidermis or dermis. In **Figure 11C**, the micro-perforators 113 and a basal layer 115 remain in contact with the skin 112 and, optionally, the remainder of the patch system is displaced from the skin. In a preferred embodiment, drug molecules in a reservoir within the upper reservoir of the patch system flow through the channel created by a fully or partially dissolved micro-perforator and into the epidermis. The drug molecules then diffuse into the dermis for local treatment or for transport through the body.

It is important that the micro-perforators in the array dissolve upon contact with (i.e., in or on) the skin. The dissolution can occur chemically, or physically (such as by melting at skin temperature), or both. Adequate dissolving of a micro-perforator is defined herein to mean that at least 50% (by weight) of the micro-perforator dissolves within four hours of administration. In many instances a faster rate of dissolution is preferred, such that at least 50% (by weight) of the micro-perforator dissolves within two hours, one hour, 30 minutes, or even 10 minutes of administration. Rapid dissolution is defined herein to mean that at least 50% (by weight) of the micro-perforator dissolves within one hour of administration.

Micro-perforators are deemed "substantially solid" as long as they are sufficiently solid to pierce the skin to a degree needed to administer a drug contained in the perforators.

Preferred patch systems include a reservoir containing a liquid or gel form of the second drug, and one or more micro-perforators extending from at least a part of the reservoir's surface. The micro-perforators associated with the patch system penetrate the stratum corneum of the skin to enhance percutaneous drug administration and to provide prompt drug delivery and/or prompt drug cut off. In the patch system, the micro-perforators and the reservoir can be constructed as a single unit or as separate units.

The patch reservoir is intended to provide sustained, controllable delivery of a liquid or semi-liquid (second) drug into or across a biological barrier so that diffusion channels are created and remain open after insertion and dissolution of a micro-perforator (matrix plus drug). The size, shape, composition and areal density of micro-perforators affect the drug release rate through the skin channels. Alternatively, other control mechanisms such as iontophoresis, sonophoresis, heating components and mechanical vibration forces can accelerate, decelerate or otherwise control drug transport through the stratum corneum.

Patch systems are preferably applied to the skin so that one or more micro-perforators penetrate through the stratum corneum, into the epidermis or into the dermis depending on the application. In a preferred embodiment, drug molecules in a reservoir in a patch system flow through the channel created by a fully or partially dissolved micro-perforator and into the epidermis or dermis. The drug molecules diffuse into the dermis for local treatment or for transport through the body.

Patch and other RDMP systems can transport a wide range of therapeutic and/or prophylactic agents, including drugs and vaccine and other bioactive molecules, across skin and other tissues. Such systems permit drug delivery and access to body fluids across skin or other tissue barriers, with minimal damage, pain and/or irritation at the tissue. In drug delivery applications, a micro-perforator can advantageously comprise primarily an active drug and a dissolving or swelling solid matrix depending on a desired drug profile. That acts as both an immediate drug source and as a channel creator for subsequent drug delivery through skin. In a diagnostic application, micro-perforators would generally contain no drug, and be comprised substantially entirely of a fast-dissolving, biologically inactive solid, or a dissolvable or swellable matrix for creating and maintaining the channels. Depending on the application, an osmotically active or anti-irritant compound can have a beneficial effect. In some diagnostic applications, it is contemplated that micro-perforators can include or consist of sensor materials that react to the presence of specific analytes.

A primary function of a basal layer in a reservoir system is to separate the drug reservoir, and to serve as a transport bridge between the reservoir and the remainder of the system. The basal layer material can be same as the solid matrix material, or may be a different material, depending on the application. In a RDMP system without a reservoir, the basal layer can be laminated with one or more additional layers or materials for controlled release. The outermost basal layer can act as an impermeable backing film to protect against any virus or bacterium that might otherwise invade the skin perforation region. In order to avoid back diffusion from other parts of the RDMP system, this layer should have low drug solubility. Where additional and sustained drug release is required, the basal layer can be constructed to contain more of a drug or to provide a conduit to a secondary reservoir. It is useful to have anti-virus and/or anti-bacterial protection in the basal layer to suppress infection. In order to vary or control the drug delivery rate, an external physical enhancement system, using iontophoresis, or sonophoresis, piezoelectric response or a similar response, can be provided as part of a basal layer and/or an overlay layer.

**Figure 12A** illustrates a simple design of a patch 120A, including a basal layer 121including a backing and an array of one or more micro-perforators 122 located adjacent to the skin 123. Active ingredients (drug or drug solid solution) are contained in the micro-perforator. The basal layer can have different thicknesses but is most often an impermeable backing layer. This design is ideal for potent drug delivery, for administrating small doses or for instant drug delivery.

**Figure 12B** illustrates another design of a patch 120B, including an array of micro-perforators 122 and a basal layer 124. Active ingredients are contained in the micro-perforator and in the basal layer. The basal layer 124 can have different composition from the Micro-perforator(s) outermost layer but is most often an impermeable backing layer. This design is also ideal for potent drug delivery, for administrating small dose systemically, or for topical applications.

**Figure 12C** illustrates a more complex design of a patch 120C. Active ingredients are contained in the micro-perforator and in the reservoir. A top portion of the basal layer 126 contains a drug reservoir patch 125. A first array of micro-perforators 122-1 is oriented to penetrate the stratum corneum of the skin 123, and a second array of micro-perforators 122-2 is oppositely oriented to penetrate a membrane that surrounds or contains fluid in the reservoir patch 125. The reservoir patch 125 can be applied after application of the arrays, or can be combined with the arrays. The reservoir can have a variety of composition or formulation depending on targeted drug release profile.

**Figures 12D and 12E** illustrate other designs, 120D and 120E, of RDMP patch systems modified from Figures 12B and 12C, respectively. Here, the micro-perforators do not contain any drug, but do include a dissolving matrix. The active ingredients are contained in the basal layer and/or reservoir only. Depending on the drug solubility and concentration, drug release from the basal layer can be controlled. The systems shown in Figures 12D and 12E are used primarily for diagnostic purposes and for sustained drug release applications, respectively.

**Figure 12F** illustrates use of swellable materials as part of a patch system 120F, optionally with no drug(s) included in the micro-perforator solid matrix. This design extends skin channel openings for a longer (or shorter) time interval and extends the duration of drug delivery or is used for diagnostic applications.

Any drug or other bioactive agent can be delivered using a RDMP system. Delivered drugs can be can be proteins, peptides, DNA, genes, polysaccharides, and synthetic organic and inorganic compounds. Representative agents include, but are not limited to, antiinfectives, hormones, growth regulators, drugs regulating cardiac action or blood flow, and drugs for pain control. The drug can be for local treatment or for regional or systemic therapy. The following are representative protein drug examples dose per injection they are used to treat:

| | |
|---|---|
| α-interferon | 11 -100 *µ*gm |
| β-interferon for multiple sclerosis | 22 - 44 *µ*gm |

| | |
|---|---|
| Erythropoietin for anemia | 10 - 30 *µ*gm |
| Follicle stimulating hormone (FSH) | 5 - 30 *µ*gm |
| G-CSF | 9 - 15 *µ*gm |
| GM - CSF | 250 *µ*gm |
| Human chorionic gonadotropin | 30 - 300 *µ*gm |
| Leutinizing hormone | 2-30 *µ*gm |
| Salmon Calcitonin | 25 - 50 *µ*gm |
| Glucagon | 1 mgm |
| GNRH antagonist | 2 mgm |
| Insulin | 0.75 - 1.5 mgm |
| Human Growth Hormone (GHD) | 0.25 - 1.5 mgm |
| Human Growth Hormone (AIDS) | 6 mgm |
| Testosterone | 5 - 10 mgm |
| Lidocaine | 2 - 5 % |
| Diclofenac Sodium | 100 200 mgm |
| Oxybutynin | 5 -15 mgm |
| Ketoprofen | 75 - 200 mgm |
| Alemdronate | 10 mgm |
| Enalpril Maleate | 10 - 40 mgm |
| Phenylpropanolamine HCl | 75 mgm |
| Cromolyn sodium | 3.2 - 10 mgm |
| Isotretinoin | 0.5 - 2 mgm/Kgm |
| Oxytocin | 1-2 unit/min/iv |
| Paroxetine HCl | 20 mgm |
| Flurbiprofen | 100 mgm |
| Sertaline | 50 mgm |
| Venlafaxine | 75 mgm |
| Leuprolide | 0.125 - 0.25 mgm |
| Risperidone | 4 - 6 mgm |
| Galanthamine hydrobromide | 16 - 24 mgm |
| Enoxaprin, anticoagulant | |
| Etanercept, rheumatoid arthritis | |
| Fentanyl, postoperative and chronic pain | |
| Filgrastin, low white blood cells from | |
| chemotherapy | |
| Heparin, anticoagulant | |
| Parathyroid hormone (PTH) | |
| Somatropin, growth hormone | |
| Sumatriptan, migraine headaches | |
| Morphine | |
| Opiate anti-arthritis. | |

Drugs can be delivered at a variety of therapeutic rates, controlled by varying a number of design factors including: dimensions of the array, dissolving rate of the matrix, number and distribution of micro-perforators in the array, size of the patch, size and composition of the reservoir, and frequency of using the arrays. For example, devices designed to deliver drug at high rates might have a more active drug in the micro-perforators, and/or a faster dissolving matrix. For sustained drug release, fewer micro-perforators and/or use of a slow(er) dissolving solid matrix are useful. The patch can be applied to the skin or other tissue to deliver drugs continuously or intermittently or at a varying rate, for time intervals ranging from a few seconds to several hours or days. Most applications of RDMP drug transdermal delivery would target the epidermis, although delivery into blood stream directly is available by extending the penetration length of a patch.

The RDMP systems disclosed herein are also useful for controlling transport across tissues other than skin. For example, a patch can be inserted into a patient's eye to control or correct conjunctiva, sclera, and/or cornea problems, to facilitate delivery of drugs into the eye with a slow moving actuator. Similarly, a RDMP array inserted into the eye could facilitate transport of fluid out of the eye, which may be of benefit for treatment of glaucoma. A RDMP patch can also be inserted into the buccal (oral), nasal or vaginal regions or inside a tissue with the aid of a laparoscope or into other accessible mucosal layers to facilitate transport into or across those tissues. For example, a drug may be delivered across the buccal mucosal for local treatment in the mouth. As another example, RDMP arrays may be used internally within the body on, for example, the lining of the gastrointestinal tract to facilitate uptake of orally ingested drugs or at the lining of blood vessels to facilitate penetration of drugs into the vessel wall. In this case of internal tissue application, use of bio-adhesive RDMP material can be an additional benefit.

Another important application is vaccination. The skin is an ideal site for effective vaccine delivery because it contains a network of immune cells, such as Langerhans cells. There are several advantages of RDMP technology in delivering antigenic compounds to the epidermis, which has a high density of immune cells and consequently triggers the immune system more effectively. A RDMP system is a practical design for developing multivalent vaccines easily and is expected to provide more stability than use of a liquid for transport and storage of drugs. The following vaccines can be delivered, among others.
Hepatitis B
HIV vaccine
Influenza
Diphtheria
Tetanus
Pertussis
Lyme disease
Rabies
Pneumococcus
Yellow fever
Cholera
Vaccinia
Tuberculosis
Rubella
Measles
Mumps
Rotavirus
Botulinum
Herpes virus
Other DNA vaccines
Hepatitis B
HIV vaccine
Influenza
Diphtheria
Tetanus
Pertussis
Lyme disease
Rabies
Pneumococcus
Yellow fever
Cholera
Vaccinia
Tuberculosis

Another area of applications is cosmeceutical. A RDMP system including a patch can deliver botox toxin or a hydroxyacid more efficiently and safely to remove or reduce wrinkle formation and skin aging. The system is also useful for treating lesions or abnormal skin features, such as pimples, corns, warts, calluses, bunions, actinic keratoses and hard hyperkeratotic skin, which is often found on the face, arms, legs or feet. A RDMP system is also useful as a food patch to deliver essential amino acids, fats and vitamins. A food patch is often used in emergencies.

Thus, specific embodiments and applications of rapidly dissolving micro-perforators have been disclosed. It should be apparent, however, to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

## Claims

1. Skin patch device (120A-F) for delivering drugs percutaneously, comprising
rapidly dissolving micro-perforators (41, 51, 61, 71, 81, 91, 113, 122), wherein
the micro-perforators (41, 51, 61, 71, 81, 91, 113, 122) comprising
a solid matrix of dissolvable material that holds one or more selected drugs, such that, when body fluid and/or solvent contacts the micro-perforators (41, 51, 61, 71, 81, 91, 113, 122), the micro-perforators (41, 51, 61, 71, 81, 91, 113, 122) rapidly dissolve.

2. Skin patch device (120A-F) according to claim 1, wherein
the drug(s) is/are contained within a polymeric matrix material.

3. Skin patch device (120A-F) according to claim 1, wherein
the drug(s) comprising substantially 100% of the micro-perforator(s) (41, 51, 61, 71, 81, 91, 113, 122).

4. Skin patch device (120A-F) according to claim 1, wherein
the drug(s) comprising a peptide or a nucleic acid.

5. Skin patch device (120A-F) according to claim 1, wherein
the drug(s) comprising a polysaccharide.

6. Skin patch device (120A-F) according to claim 1, wherein
the drug(s) comprising a vaccine.

7. Skin patch device (120A-F) according to claim 1, wherein
the micro-perforators (41, 51, 61, 71, 81, 91, 113, 122) are sufficiently long to penetrate the skin (11, 42, 52, 62, 72, 81, 92, 112, 123) to the dermal layer (17).

8. Skin patch device (120A-F) according to claim 1, wherein
the micro-perforators (41, 51, 61, 71, 81, 91, 113, 122) have a substantially polygonal cross-section.

9. Skin patch device (120A-F) according to claim 1, wherein
the micro-perforators (41, 51, 61, 71, 81, 91, 113, 122) have a substantially cusp-shaped cross-section.

10. Skin patch device (120A-F) according to claim 1, wherein
the micro-perforators (41, 51, 61, 71, 81, 91, 113, 122) are substantially blade shaped.

11. Skin patch device (120A-F) according to claim 1, wherein
the micro-perforators (41, 51, 61, 71, 81, 91, 113, 122) have a minimum tip diameter no more than 20 µm.

12. Skin patch device (120A-F) according to claim 1, wherein
at least 5 of the micro-perforators (41, 51, 61, 71, 81, 91, 113, 122) are disposed in a region having an area no more than 1 cm².

13. Skin patch device (120A-F) according to claim 1, wherein
the source of solvent comprises a fluid
reservoir containing a second drug.

14. Skin patch device (120A-F) according to claim 13, wherein the reservoir comprises a patch (125).

15. Skin patch device (120A-F) according to claim 14, wherein the drug contained in the micro-perforators (41, 51, 61, 71, 81, 91, 113, 122) is the same as the drug contained in the patch (125).

16. Skin patch device (120A-F) according to claim 14, wherein the drugs contained in the micro-perforators (41, 51, 61, 71, 81, 91, 113, 122) and the drug contained in the patch (125) are different from each other.

17. Skin patch device (120A-F) according to claim 14, wherein the patch (125) has a peripheral region of adhesive.

18. Skin patch device (120A-F) according to claim 1, wherein
the source of solvent includes a chemical enhancer drawn from a group consisting of an alcohol, an amide, an amino acid, a phospholipid, a sulfone, a pyrrolidone and a surfactant.

19. Skin patch device (120A-F) according to claim 1, further comprising
a second array of micro-perforators (41, 51, 61, 71, 81, 91, 113, 122) that contain a second drug.

20. Skin patch device (120A-F) according to claim 1, further comprising
a manually operated pressure mechanism that drives the micro-perforators (41, 51, 61, 71, 81, 91, 113, 122) into the skin (11, 42, 52, 62, 72, 81, 92, 112, 123).

21. Skin patch device (120A-F) according to claim 1, further comprising
a pressure mechanism that drives the micro-perforators (41, 51, 61, 71, 81, 91, 113, 122) into the skin (11, 42, 52, 62, 72, 81, 92, 112, 123) a distance of 10-1000 µm.

## Patentansprüche

1. Hautpflastervorrichtung (120A-F) zum perkutanen Abgeben von Medikamenten, aufweisend
schnellauflösende Mikro-Perforatoren (41, 51, 61, 71, 81, 91, 113, 122), wobei
die Mikro-Perforatoren (41, 51, 61, 71, 81, 91, 113, 122) aufweisen
eine feste Matrix aus lösbarem Material, das ein oder mehrere ausgewählte Medikamente enthält, so dass, wenn Körperflüssigkeit und/oder ein Lösungsmittel mit den Mikro-Perforatoren (41, 51, 61, 71, 81, 91, 113, 122) in Kontakt gerät, sich die Mikro-Perforatoren (41, 51, 61, 71, 81, 91, 113, 122) schnell auflösen.

2. Hautpflastervorrichtung (120A-F) gemäß Anspruch 1, wobei
das/die Medikament(e) innerhalb eines polymerischen Matrixmaterials enthalten ist/sind.

3. Hautpflastervorrichtung (120A-F) gemäß Anspruch 1, wobei
das/die Medikament(e) im Wesentlichen 100% des/der Mikroperforators/Mikroperforatoren (41, 51, 61, 71, 81, 91, 113, 122) umfasst/umfassen.

4. Hautpflastervorrichtung (120A-F) gemäß Anspruch 1, wobei das/die Medikament(e) ein Peptid oder eine Nukleinsäure aufweist/aufweisen.

5. Hautpflastervorrichtung (120A-F) gemäß Anspruch 1, wobei das/die Medikament(e) ein Polysaccharid aufweist/aufweisen.

6. Hautpflastervorrichtung (120A-F) gemäß Anspruch 1, wobei
das/die Medikament(e) einen Impfstoff aufweist/ aufweisen.

7. Hautpflastervorrichtung (120A-F) gemäß Anspruch 1, wobei
die Mikro-Perforatoren (41, 51, 61, 71, 81, 91, 113, 122) ausreichend lang sind, um die Haut (11, 42, 52, 62, 72, 81, 92, 112, 123) zu der Dermalschicht (17) zu durchdringen.

8. Hautpflastervorrichtung (120A-F) gemäß Anspruch 1, wobei
die Mikro-Perforatoren (41, 51, 61, 71, 81, 91, 113, 122) einen im Wesentlichen polygonalen Querschnitt aufweisen.

9. Hautpflastervorrichtung (120A-F) gemäß Anspruch 1, wobei
die Mikro-Perforatoren (41, 51, 61, 71, 81, 91, 113, 122) einen im Wesentlichen spitz geformten Querschnitt aufweisen.

10. Hautpflastervorrichtung (120A-F) gemäß Anspruch 1, wobei
die Mikro-Perforatoren (41, 51, 61, 71, 81, 91, 113, 122) im Wesentlichen klingenförmig sind.

11. Hautpflastervorrichtung (120A-F) gemäß Anspruch 1, wobei
die Mikro-Perforatoren (41, 51, 61, 71, 81, 91, 113, 122) einen minimalen Spitzendurchmesser aufweisen, der nicht größer ist als 20 µm.

12. Hautpflastervorrichtung (120A-F) gemäß Anspruch 1, wobei
wenigstens 5 der Mikro-Perforatoren (41, 51, 61, 71, 81, 91, 113, 122) in einem Bereich angeordnet sind, der nicht größer ist als 1 cm².

13. Hautpflastervorrichtung (120A-F) gemäß Anspruch 1, wobei
die Quelle des Lösungsmittels ein Flüssigkeitsreservoir aufweist, das ein zweites Medikament enthält.

14. Hautpflastervorrichtung (120A-F) gemäß Anspruch 13, wobei das Reservoir ein Pflaster (125) aufweist.

15. Hautpflastervorrichtung (120A-F) gemäß Anspruch 14, wobei das Medikament, das in den Mikro-Perforatoren (41, 51, 61, 71, 81, 91, 113, 122) enthalten ist, das Gleiche ist wie das Medikament, das in dem Pflaster (125) enthalten ist.

16. Hautpflastervorrichtung (120A-F) gemäß Anspruch 14, wobei das Medikament, das in den Mikro-Perforatoren (41, 51, 61, 71, 81, 91, 113, 122) enthalten ist, und das Medikament, das in dem Pflaster (125) enthalten ist, unterschiedlich sind.

17. Hautpflastervorrichtung (120A-F) gemäß Anspruch 14, wobei das Pflaster (125) einen Umfangsbereich aus Klebstoff aufweist.

18. Hautpflastervorrichtung (120A-F) gemäß Anspruch 1, wobei
die Quelle des Lösungsmittels einen chemischen Beschleuniger aufweist, bezogen aus einer Gruppe, welche besteht aus einem Alkohol, einem Amid, einer Aminosäure, einem Phospholipid, einem Sulfon, einem Pyrrolidon und einer oberflächenaktiven Substanz.

19. Hautpflastervorrichtung (120A-F) gemäß Anspruch 1, des Weiteren aufweisend
einen zweiten Bereich aus Mikro-Perforatoren (41, 51, 61, 71, 81, 91, 113, 122), die ein zweites Medikament enthalten.

20. Hautpflastervorrichtung (120A-F) gemäß Anspruch 1, des Weiteren aufweisend
einen manuell bedienbaren Druckmechanismus, der die Mikro-Perforatoren (41, 51, 61, 71, 81, 91, 113, 122) in die Haut (11, 42, 52, 62, 72, 81, 92, 112, 123) treibt.

21. Hautpflastervorrichtung (120A-F) gemäß Anspruch 1, des Weiteren aufweisend
einen Druckmechanismus, der die Mikro-Perforatoren (41, 51, 61, 71, 81, 91, 113, 122) in die Haut (11, 42, 52, 62, 72, 81, 92, 112, 123) treibt, mit einer Wegstrecke von 10-1000 µm.

## Revendications

1. Dispositif formant emplâtre (120A à 120F) destiné à administrer des médicaments de manière percutanée, comprenant
des micro-perforateurs à dissolution rapide (41, 51, 61, 71, 81, 91, 113, 122), dans lequel les micro-perforateurs (41, 51, 61, 71, 81, 91, 113, 122) comprennent :
une matrice solide de matériau pouvant être dissous qui contient un ou plusieurs médicaments sélectionnés, de telle sorte que, lorsqu'un fluide corporel et/ou un solvant entre en contact avec les micro-perforateurs (41, 51, 61, 71, 81, 91, 113, 122), les micro-perforateurs (41, 51, 61, 71, 81, 91, 113, 122) se dissolvent rapidement.

2. Dispositif formant emplâtre (120A à 120F) selon la revendication 1, dans lequel le ou les médicaments sont contenus à l'intérieur d'un matériau en une matrice polymère.

3. Dispositif formant emplâtre (120A à 120F) selon la revendication 1, dans lequel le ou les médicaments comprennent sensiblement 100% des micro-perforateurs (41, 51, 61, 71, 81, 91, 113, 122).

4. Dispositif formant emplâtre (120A à 120F) selon la revendication 1, dans lequel le ou les médicaments comprennent un peptide ou un acide nucléique.

5. Dispositif formant emplâtre (120A à 120F) selon la revendication 1, dans lequel le ou les médicaments comprennent un polysaccharide.

6. Dispositif formant emplâtre (120A à 120F) selon la revendication 1, dans lequel le ou les médicaments comprennent un vaccin.

7. Dispositif formant emplâtre (120A à 120F) selon la revendication 1, dans lequel
les micro-perforateurs (41, 51, 61, 71, 81, 91, 113, 122) sont suffisamment longs pour pénétrer dans la peau (11, 42, 52, 62, 72, 81, 92, 112, 123) jusqu'à la couche dermique (17).

8. Dispositif formant emplâtre (120A à 120F) selon la revendication 1, dans lequel
les micro-perforateurs (41, 51, 61, 71, 81, 91, 113, 122) présentent une section transversale sensiblement polygonale.

9. Dispositif formant emplâtre (120A à 120F) selon la revendication 1, dans lequel
les micro-perforateurs (41, 51, 61, 71, 81, 91, 113, 122) présentent une section transversale sensiblement en forme de pointe acérée.

10. Dispositif formant emplâtre (120A à 120F) selon la revendication 1, dans lequel
les micro-perforateurs (41, 51, 61, 71, 81, 91, 113, 122) sont sensiblement en forme de lame.

11. Dispositif formant emplâtre (120A à 120F) selon la revendication 1, dans lequel
les micro-perforateurs (41, 51, 61, 71, 81, 91, 113, 122) présentent un diamètre de pointe minimum non supérieur à 20 µm.

12. Dispositif formant emplâtre (120A à 120F) selon la revendication 1, dans lequel
au moins 5 des micro-perforateurs (41, 51, 61, 71, 81, 91, 113, 122) sont disposés dans une zone présentant une surface non supérieure à 1 cm2.

13. Dispositif formant emplâtre (120A à 120F) selon la revendication 1, dans lequel
la source de solvant comprend un réservoir de fluide contenant un second médicament.

14. Dispositif formant emplâtre (120A à 120F) selon la revendication 13, dans lequel le réservoir comprend un timbre (125).

15. Dispositif formant emplâtre (120A à 120F) selon la revendication 14, dans lequel le médicament contenu dans les micro-perforateurs (41, 51, 61, 71, 81, 91, 113, 122) est le même que le médicament contenu dans le timbre (125).

16. Dispositif formant emplâtre (120A à 120F) selon la revendication 14, dans lequel les médicaments contenus dans les micro-perforateurs (41, 51, 61, 71, 81, 91, 113, 122) et le médicament contenu dans le timbre (125) sont différents les uns des autres.

17. Dispositif formant emplâtre (120A à 120F) selon la revendication 14, dans lequel le timbre (125) présente une zone périphérique adhésive.

18. Dispositif formant emplâtre (120A à 120F) selon la revendication 1, dans lequel
la source de solvant comporte un agent d'amélioration chimique extrait du groupe constitué par un alcool, une amide, un acide aminé, un phospholipide, un sulfone, une pyrrolidone et un surfactant.

19. Dispositif formant emplâtre (120A à 120F) selon la revendication 1, comprenant, en outre:
une seconde matrice de micro-perforateurs (41, 51, 61, 71, 81, 91, 113, 122) qui contient un second médicament.

20. Dispositif formant emplâtre (120A à 120F) selon la revendication 1, comprenant, en outre,
un mécanisme de pression commandé manuellement qui entraîne les micro-perforateurs (41, 51, 61, 71, 81, 91, 113, 122) dans la peau (11, 42, 52, 62, 72, 81, 92, 112, 123).

21. Dispositif formant emplâtre (120A à 120F) selon la revendication 1, comprenant, en outre, un mécanisme de pression qui entraîne les micro-perforateurs (41, 51,61,71,81,91, 113, 122) dans la peau (11, 42, 52, 62, 72, 81, 92, 112, 123) sur une distance de 10 à 1000 µm.
